# EUROPEAN PATENT SPECIFICATION

(11) **EP 2 585 430 B1**
(45) Date of publication and mention of the grant of the patent: **17.04.2019**
(21) Application number: 11729657.4
(22) Date of filing: 22.06.2011
(51) Int. Cl.: C07C 51/43, C07C 63/24, C07C 63/26

(54) **Process and system for the separation of carboxylic acids (such as terephthalic acid) from a slurry**
Verfahren und System zur Abscheidung von Carboxylsäuren (wie Terephthalsäure) aus einer Aufschlämmung
Procédé et système pour la séparation d'acides carboxyliques (tels que l'acide téréphtalique) d'une suspension

(30) Priority: 28.06.2010 GB 201010857; 28.06.2010 GB 201010856
(43) Date of publication of application: 01.05.2013
(73) Proprietor: Johnson Matthey Davy Technologies Limited, London EC4A 4AB (GB)
(72) Inventor: GRAY, Julian Stuart, London W2 6LG (GB); WINTER, Michael William, London W2 6LG (GB); GNAGNETTI, Andrea, I-20151 Milan (IT)
(74) Representative: Bown, Mark Richard
(86) International application number: PCT/GB2011/051170
(87) International publication number: WO 2012/001389

(56) References cited:
- WO-A2-2009/024872
- US-A- 5 583 254
- US-A1- 2002 003 117

## Description

The present invention relates to a process for the separation of carboxylic acid crystals, preferably aromatic carboxylic acid crystals, from a solvent and a system for said separation. More particularly, it relates to a process for the separation of terephthalic acid crystals from a solvent and a system for said separation. Where the terephthalic acid crystals are crude terephthalic acid crystals they will generally be separated from acetic acid. Where the terephthalic acid crystals are pure terephthalic acid crystals they will generally be separated from water. The present invention also relates to a process for the separation of isophthalic acid crystals from a solvent and a system for said separation.

Typically crude terephthalic acid is produced by the oxidation ofp-xylene. The oxidation is conducted using acetic acid as solvent in the presence of a catalyst. The solution is then cooled in a stepwise manner to crystallise the terephthalic acid. The terephthalic acid must then be removed from the acetic acid solvent and this is commonly carried out using a rotary vacuum filter, followed by a drying step to remove residual moisture. An example of a rotary vacuum filter is described in US2002/0003117. As discussed in more detail below, gas is blown through the filter cake in the vacuum filter and this gas leaves the filter with the filtrate,

In order to prevent the filtrate from flashing as it passes through the filter cake and cloth the filter operates at a relatively low temperature. For crude terephthalic acid the temperature is of the order of 90°C.

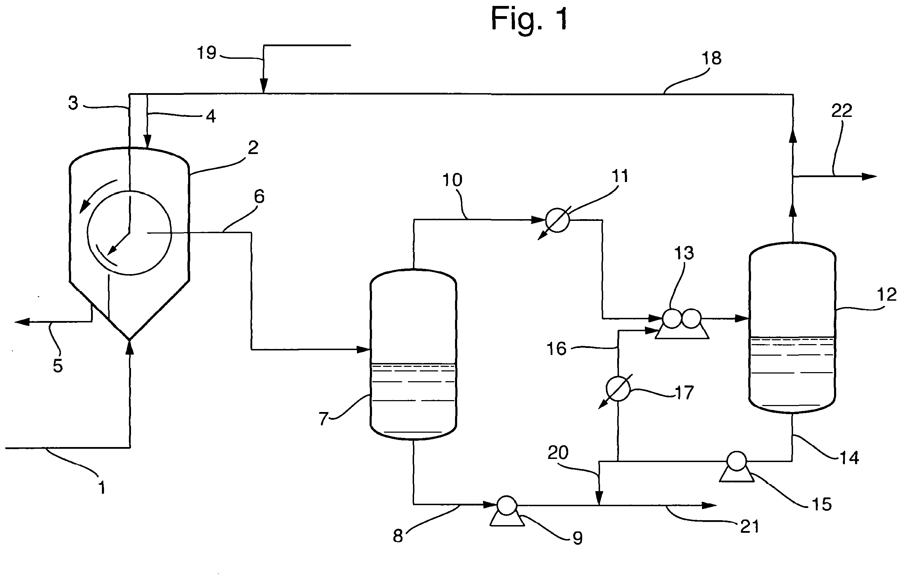

Typically, the gas leaving the filter is first cooled to condense the acetic acid vapour that has evaporated into the gas from the filtrate. The gas is then separated from the condensed acetic acid. The flow scheme for the filter and the associated gas is illustrated in Figure 1.

In the illustrated arrangement, the slurry of crude terephthalic acid in acetic acid is fed in line 1 to a rotary vacuum filter 2. Blowback gas and casing gas are supplied to the rotary vacuum filter 2 in lines 3 and 4 respectively. The wet cake is then removed in line 5.

The filtrate and gas are removed in line 6 and are passed to a gas/liquid separator 7. The separated filtrate is removed in line 8 via pump 9. The gas is removed in line 10 and cooled in condenser 11 before being passed to second gas/liquid separator 12 via vacuum pump 13. The liquid removed from the second gas/liquid separator 12 in line 14 is pumped via pump 15 and a portion is combined via line 20 with the filtrate leaving the system in line 8 and the combined stream is removed in line 21. The remainder is circulated in line 16 via the liquid ring cooler 17 and the vacuum pump 13 to the second gas/liquid separator 12. The gas from the second gas/liquid separator 12 is returned to the rotary vacuum filter 2 via line 18 where it is split with a portion of the gas supplied as casing gas in line 4 and the remainder as blowback gas in line 3. Make up gas may be added in line 19 and a purge may be taken in line 22. The motive driving force for the circulating system is generally provided by the liquid ring vacuum pump 13.

The gas used in the system is generally an inert gas and will generally comprise nitrogen. Other components, such as oxygen and carbon dioxide may also be present.

A similar process may be used to separate pure terephthalic acid from water. Here the separation may occur in two stages with the first occurring in a centrifuge and the second in a rotary vacuum filter. In this second, the rotary vacuum filter stage will have the same gas cycle scheme as detailed above.

The amount of gas flowing through the filter cake and filter cloth is significant. The use of pure nitrogen would be very expensive and thus typically the gas is provided from the reactor in which the crude terephthalic acid is formed. The gas removed from the reactor is cleaned up to remove carbon monoxide and organic compounds before being passed to the rotary vacuum filter. If the reactor offgas were not used in this manner, it would conventionally be expanded in the offgas expander and thereby generate power. Thus each amount of offgas removed for use in the rotary vacuum filter represents a loss of power generated which affects the efficiency of the system. There is therefore an economic driver to reduce the consumption of inert gas to a minimum to maximise the amount available to generate power.

In order to meet the requirement to minimise the amount of gas required the recirculation system detailed above is used. This is the most economic way of providing the required flow of inert gas to partially dry the wet cake after washing.

However, this recirculation gas system requires several items of equipment to be provided including a separator vessel, condenser, vacuum pumps, and associated piping. Since a plant will generally include more than one rotary vacuum filter (two are conventionally found in a typical pure terephthalic acid plant) the equipment for the recycle system must be provided for each rotary vacuum filter. Thus the presence of the conventional recirculation system adds significantly to the capital costs of the plant.

This problem is exacerbated as vacuum pumps are expensive. In addition, the operation of the vacuum filter and the gas recirculation system have high operating costs since the vacuum pump consumes a significant amount of electricity.

A further problem is that the vacuum pumps tend to be unreliable. The problem is exacerbated as the pump has to be stopped and restarted every time the filter is washed which can be at least once or twice a day.

An alternative process for the production of purified terephthalic acid is described in WO 93/24440. In this process an aqueous solution of crude terephthalic acid is subjected to hydrogenation to reduce impurities. The resulting solution is then crystallised to produce a slurry of purified terephthalic acid in aqueous liquor. An integrated terephthalic acid separation and washing process is then carried out at elevated pressure by means of a belt filtration system. However, this arrangement still requires a recirculation system for the inert gas; it is therefore apparent that appreciable volumes of gas are required.

WO2009/024872 describes a method for the recovery of carboxylic acid comprising the steps of introducing a liquid slurry comprising water, crystallized carboxylic acid such as terephthalic acid, isophthalic acid, trimellitic acid, and impurities into a high pressure rotary filter; filtering said slurry and collecting at least some of the solid portion; passing the collected solid portion to a rotary valve comprising a temperature control system; controlling the temperature in the rotary valve through the temperature control system; and removing the solid portion from the rotary valve. The rotary valve comprises a valve case and a rotor, and the temperature control system controls the temperature difference between the valve case and the rotor. A recirculation gas system is used.

An alternative method in which a recirculation gas system is used is described in US5583254. In this method purified terephthalic acid is prepared by subjecting an aqueous solution of crude terephthalic acid to hydrogenation to reduce impurities, crystallizing this solution to produce a slurry of purified terephthalic acid in an aqueous liquor, and carrying out an integrated separation and washing process. The integrated separation is performed by exchanging the acidic reaction medium with water to produce a water containing cake of terephthalic acid.

It is therefore desirable to reduce the gas consumption of the filter such that the recirculation gas system is not required.

According to the present invention there is provided a process for the separation of terephthalic acid or isophthalic acid from a slurry in a solvent comprising the steps of:
supplying the slurry comprising crystals of terephthalic acid or isophthalic acid to a filter operating at pressure and at a temperature above the atmospheric boiling point of the solvent;
mixing inert gas with solvent wherein the mixture comprises from 50 wt% inert gas and 50 wt% solvent to 10 wt% inert gas and 90 wt% solvent;
supplying said inert gas and solvent to the filter; and
removing a cake of separated crystals;
wherein the inert gas removed from the filter is not recycled.

According to a second aspect of the present invention, not claimed herein, there is provided a system for the separation of carboxylic acid crystals from a slurry in a solvent comprising:
a pressure filter device having a slurry inlet, and an outlet for a cake of carboxylic acid crystals;
means for mixing inert gas and solvent wherein the mixture comprises from 50 wt% inert gas and 50 wt% solvent to 10 wt% inert gas and 90 wt% solvent;
means for supplying said inert gas and solvent to the pressure filter;
wherein said pressure filter device is configured to operate at pressure and at a temperature above the atmospheric boiling point of the solvent; and
wherein the system does not include means for recycling the inert gas removed from the filter.

The process (and system) of the present invention is particularly suitable for the separation of crude terephthalic acid from a slurry in acetic acid or the separation of pure terephthalic acid from a slurry in water. By "pure terephthalic acid" we mean terephthalic acid which has been subjected to at least one purification process and as such is more pure than the crude terephthalic acid removed from the reactor in which it is formed. It is also suitable for the separation of a slurry comprising pure or crude isophthalic acid.

Any suitable pressure filter may be used with a rotary pressure filter being particularly preferred.

Since a pressure filter is used, unlike the vacuum system where the slurry for filtration has to be cooled, the pressure filter operates at a temperature above the boiling point of the solvent. Thus, for example, where the process relates to the separation of crude terephthalic acid from acetic acid, the filter will be operated at a temperature above the boiling point of acetic acid at atmospheric pressure. Similarly, where the process relates to the separation of pure terephthalic acid from water, the filter will be operated at a temperature above the boiling point of water at atmospheric pressure.

The temperature and pressure at which the filter is operated will depend on the crystals to be separated. In one arrangement, which is particularly suitable where the crystals are those of crude terephthalic acid, the temperature of the filter may be from 110°C to about 160°C and the pressure may be from 2 to 5 bara. Temperatures in the region of 135°C to 145°C may be particularly preferred. In an arrangement where the crystals to be separated are those of pure terephthalic acid, the temperature of the filter may be from about 125°C to about 160°C and the pressure may be from 2 to 5 bara. Temperatures in the region of about 145°C to about 150°C may be particularly preferred.

With the process and system of the present invention, the mixing of the inert gas with solvent reduces the amount of inert gas required such that a recirculation system is no longer required. It will therefore be understood that the process of the present invention allows the gas to be used in a "once pass" arrangement. This therefore reduces the capital and operating costs of the process and system and the problems of the prior art arrangements are addressed.

Further, since the amount of inert gas required is substantially reduced, the amount of offgas from the reactor which is required for the filter is minimised and hence the amount of gas passed to the expander to generate power can be maximised further increasing the economics of the system.

Any suitable inert gas may be used. Generally, nitrogen will be the preferred inert gas. This may be taken as a portion of the offgas from the reactor in which the carboxylic acid is produced, or it can be provided from a different source.

By "mixed" we mean that gas is mixed with the solvent before the gas passes through the filter cake. Thus the mixing can take place outside of the filter casing or inside the filter casing. In an alternative arrangement, a proportion of the total of solvent required can be mixed with the inert gas outside of the filter casing. The remainder of the required solvent may be added inside the filter casing.

Any suitable amount of solvent may be mixed with the inert gas provided that it comprises from about 50 wt% inert gas and about 50 wt% solvent to about 10 wt% inert gas and about 90 wt% solvent. In a preferred arrangement, the mixture supplied to the filter will comprise about 20 wt% inert gas and about 80 wt% solvent.

The solvent may be provided in gaseous form and is mixed with the inert gas. In one alternative arrangement, the solvent may be provided in liquid form and is evaporated once it has been mixed with the inert gas such that it is a gaseous mixture supplied to the filter. Where the solvent is added as a liquid, the heat of the inert gas may cause the solvent to vaporise. However, additional heating means may be provided if required.

The solvent mixed with the inert gas will depend on the solvent in which the crystals of carboxylic acids are slurried on addition to the filter. Generally the solvent will be the same solvent as that in which the crystals are slurried. Alternatively the solvent they may be different but will generally be mutually compatible. Thus where the slurry is of crude terephthalic acid in acetic acid, the solvent mixed with the inert gas will preferably be acetic acid. Similarly, where the slurry is of pure terephthalic acid in water, the solvent mixed with the inert gas will preferably be water.

Where the inert gas supplied is that recovered from elsewhere in the process, it may, in certain circumstances already include some of the solvent vapour required.

The present invention will now be described, by way of example, with reference to the accompanying drawings:
- Figure 1: is a schematic representation of the process and system under the prior art;
- Figure 2: is a schematic representation of one arrangement of the process and system according to the present invention;
- Figure 3: is a schematic representation of an alternative arrangement of the process and system according to the present invention; and
- Figure 4: is a schematic representation illustrating gas being provided from upstream processing

It will be understood by those skilled in the art that the drawings are diagrammatic and that further items of equipment such as reflux drums, pumps, vacuum pumps, compressors, gas recycle compressors, temperature sensors, pressure sensors, pressure relief valves, control valves, flow controllers, level controllers, holding tanks, storage tanks, and the like may be required in a commercial plant. The provision of such ancillary items of equipment forms no part of the present invention and is in accordance with conventional chemical engineering practice.

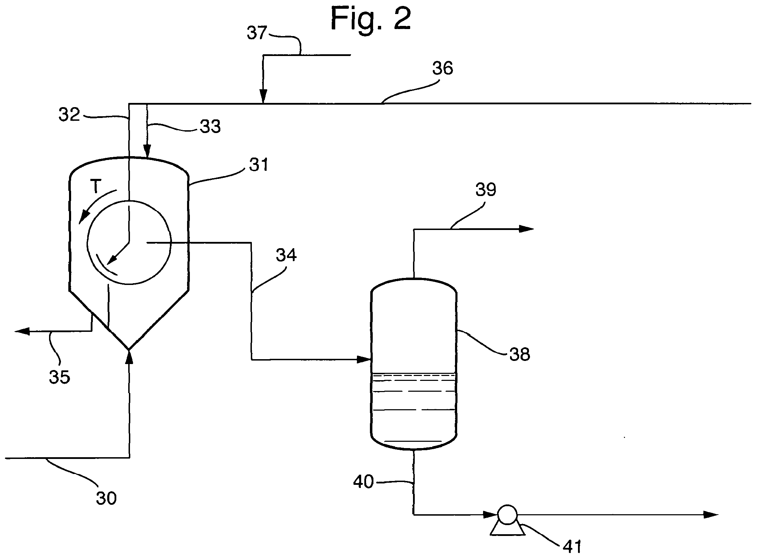

By way of example, the process and system of the present invention will be described with reference to the separation of the terephthalic acid from acetic acid. As illustrated in Figure 2, the slurry of terephthalic acid in acetic acid is passed in line 30 to a rotary pressure filter 31 where the crystals of terephthalic acid are separated from the acetic acid under pressure. Blowback gas is introduced in line 32 and casing gas is introduced in line 33. The filtrate and gas is removed in line 34. The cake of crystals of terephthalic acid are removed in line 35 and forwarded for processing. The direction of travel of the cake is illustrated by arrow T. The blowback gas of line 32 and the casing gas in line 33 are formed from inert gas supplied in line 36 which has been mixed with solvent that is supplied in line 37. Generally the solvent will be heated and vaporised before being added to the inert gas. Alternatively, the gas and solvent may be mixed before being heated to the desired temperature which will be above the boiling point of the solvent.

The filtrate and gas removed in line 34 is passed to a gas/liquid separator 38. The gas is removed as a purge in line 39 and the filtrate is removed in line 40 via pump 41.

It will therefore be seen that the equipment requirements are substantially reduced from those of the prior art arrangement illustrated in Figure 1.

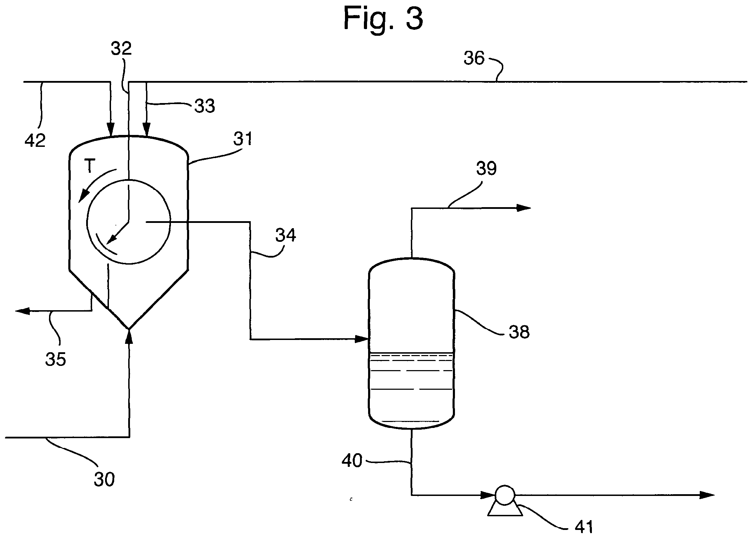

An alternative arrangement is illustrated in Figure 3. Here corresponding apparatus is identified by the same number. In this arrangement, the solvent is not added into the inert gas in line 36 but is supplied to the filter casing in line 42 such that the mixing of the solvent vapour and inert gas occurs within the casing.

It will be understood that the process may include a combination of the arrangements illustrated in Figures 2 and 3 in which solvent vapour is added to both lines 37 and 42.

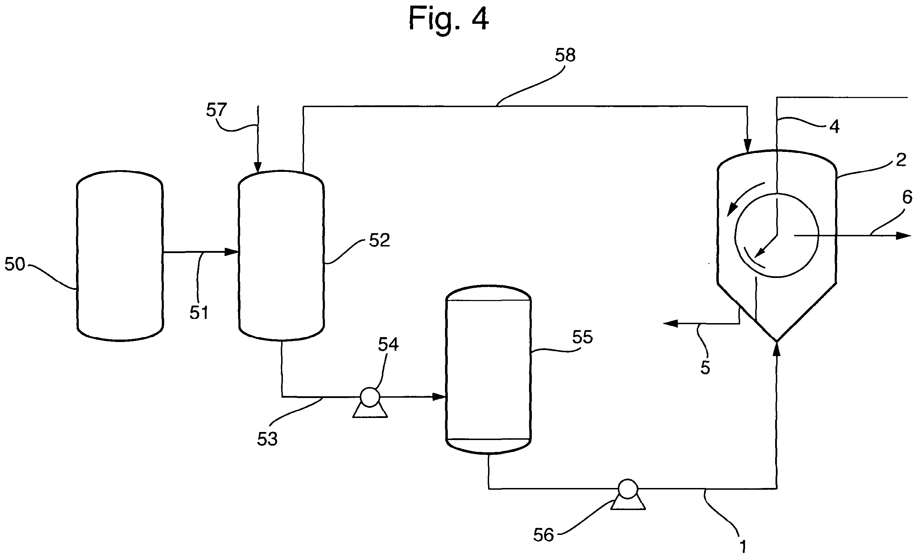

The inert gas and/or the vapour can be supplied from upstream in the reaction scheme. One example of this is illustrated in Figure 4. *p*-Xylene is oxidised in reactor 50 to produce crude terephthalic acid which is passed in line 51 to a crystallisation zone 52. The crystals are removed in line 52 via pump 54 and passed to filter feed drum 55. From there they are passed in line 1 via pump 56 to rotary pressure filter 2. The inert gas intended as casing gas can be introduced in line 57 to the crystallisation zone 52 where it picks up acetic acid vapours. The combined inert gas and acetic acid vapour is then passed in line 58 to the filter 2. It will be understood that this stream could be used additionally or alternatively as blowback gas. Similarly additional vapour and/or inert gas can be supplied as required.

### Example 1 and Comparative Example 2

A process for the preparation of terephthalic acid is prepared and the crude acid as a slurry in the acetic acid is passed in Example 1 to a system according to the present invention using a rotary pressure filter and in Comparative Example 2 to a system according to prior art using a rotary vacuum filter.

In Example 1, the filter operates at 3.5 bara with a 0.5 differential pressure. The gas flow is 7835 kg/hr at 135°C. The gas composition is 25 wt% nitrogen and 75 wt% acetic acid. The nitrogen flow required is 1950 kg/hr.

In Comparative Example 2, the filter operates at 1.1 bara with a 0.5 differential pressure. The gas flow is 7200 kg/hr at 55°C. The gas composition is 75 wt% nitrogen and 25wt% acetic acid. The nitrogen flow required is 5400 kg/hr.

It can therefore be seen that the present invention gives a 64% reduction in the required inert gas flow rate.

## Claims

1. A process for the separation of terephthalic acid or isophthalic acid from a slurry in a solvent comprising the steps of:
supplying the slurry comprising crystals of terephthalic acid or isophthalic acid to a filter operating at pressure and at a temperature above the atmospheric boiling point of the solvent;
mixing inert gas with solvent wherein the mixture comprises from 50 wt% inert gas and 50 wt% solvent to 10 wt% inert gas and 90 wt% solvent;
supplying said inert gas and solvent mix to the filter; and
removing a cake of separated crystals;
wherein the inert gas removed from the filter is not recycled.

2. A process of Claim 1 wherein the pressure filter is a rotary pressure filter.

3. A process according to Claim 1 or Claim 2 wherein the inert gas comprises nitrogen.

4. A process according to any one of Claims 1 to 3 wherein the inert gas is provided from the manufacturing process for the terephthalic acid or isophthalic acid .

5. A process according to any one of Claims 1 to 4 wherein the gas is mixed with the solvent outside of the filter casing and/or inside the filter casing.

6. A process according to any one of Claims 1 to 5 wherein the inert gas is saturated with solvent.

7. A process according to any one of Claims 1 to 6 wherein the mixture comprises from 20 wt% inert gas and 80 wt% solvent,

8. A process according to any one of Claims 1 to 7 wherein the solvent is in gaseous form.

## Patentansprüche

1. Verfahren zum Abscheiden von Theraphthalsäure oder Isophthalsäure von einer Aufschlämmung in einem Lösemittel, folgende Schritte umfassend:
Führen der Aufschlämmung, die Kristalle der Theraphthalsäure oder
Isophthalsäure umfasst, zu einem Filter, der bei Druck und einer Temperatur über dem atmosphärischen Siedepunkt des Lösemittels arbeitet;
Mischen von inertem Gas mit dem Lösemittel, wobei die Mischung von 50 Gew.-% inertes Gas und 50 Gew.-% Lösemittel bis 10 Gew.-% inertes Gas und 90 Gew.-% Lösemittel umfasst;
Führen der Mischung aus inertem Gas und Lösemittel zu dem Filter; und
Entfernen des Kuchens aus abgeschiedenen Kristallen;
wobei das aus dem Filter entfernte inerte Gas nicht wiederverwertet wird.

2. Verfahren nach Anspruch 1, wobei der Druckfilter ein Rotationsdruckfilter ist.

3. Verfahren nach Anspruch 1 oder Anspruch 2, wobei das inerte Gas Stickstoff umfasst.

4. Verfahren nach einem der Ansprüche 1 bis 3, wobei das inerte Gas aus dem Herstellungsverfahren für die Theraphthalsäure oder Isophthalsäure bereitgestellt wird.

5. Verfahren nach einem der Ansprüche 1 bis 4, wobei das Gas mit dem Lösemittel außerhalb des Filtergehäuses und/oder innerhalb des Filtergehäuses gemischt wird.

6. Verfahren nach einem der Ansprüche 1 bis 5, wobei das inerte Gas mit Lösemittel gesättigt wird.

7. Verfahren nach einem der Ansprüche 1 bis 6, wobei die Mischung von 20 Gew.-% inertes Gas und 80 Gew.-% Lösemittel umfasst.

8. Verfahren nach einem der Ansprüche 1 bis 7, wobei das Lösemittel gasförmig ist.

## Revendications

1. Procédé de séparation d'acide téréphtalique ou d'acide isophtalique d'une suspension épaisse dans un solvant comprenant les étapes de :
fourniture de la suspension épaisse comprenant des cristaux d'acide téréphtalique ou d'acide isophtalique à un filtre fonctionnant à pression et à une température au-dessus du point d'ébullition atmosphérique du solvant ;
mélange d'un gaz inerte avec un solvant, le mélange comprenant de 50 % en poids de gaz inerte et 50 % en poids de solvant à 10 % en poids de gaz inerte et 90 % en poids de solvant ;
fourniture dudit mélange de gaz inerte et de solvant au filtre ; et
enlèvement d'un gâteau de cristaux séparés ;
dans lequel le gaz inerte enlevé du filtre n'est pas recyclé.

2. Procédé selon la revendication 1, dans lequel le filtre sous pression est un filtre rotatif sous pression.

3. Procédé selon la revendication 1 ou la revendication 2, dans lequel le gaz inerte comprend l'azote.

4. Procédé selon l'une quelconque des revendications 1 à 3, dans lequel le gaz inerte est fourni du procédé de fabrication de l'acide téréphtalique ou de l'acide isophtalique.

5. Procédé selon l'une quelconque des revendications 1 à 4, dans lequel le gaz est mélangé avec le solvant à l'extérieur du boîtier du filtre et/ou à l'intérieur du boîtier du filtre.

6. Procédé selon l'une quelconque des revendications 1 à 5, dans lequel le gaz inerte est saturé de solvant.

7. Procédé selon l'une quelconque des revendications 1 à 6, dans lequel le mélange comprend de 20 % en poids de gaz inerte et 80 % en poids de solvant.

8. Procédé selon l'une quelconque des revendications 1 à 7, dans lequel le solvant est sous forme gazeuse.
